(19) Europäisches Patentamt
European Patent Office
Office européen des brevets

(11) EP 4 355 216 B1

(12) EUROPEAN PATENT SPECIFICATION

(45) Date of publication and mention of the grant of the patent:
**26.02.2025 Bulletin 2025/09**

(21) Application number: **21769641.8**

(22) Date of filing: **27.08.2021**

(51) International Patent Classification (IPC):
***A61B 6/00*** (2024.01) ***A61B 6/03*** (2006.01)
***A61B 6/58*** (2024.01)

(52) Cooperative Patent Classification (CPC):
**A61B 6/583;** A61B 6/032

(86) International application number:
**PCT/CN2021/115082**

(87) International publication number:
**WO 2023/000437 (26.01.2023 Gazette 2023/04)**

(54) **METHOD FOR DETERMINING LONGITUDINAL POSITION OF COMBINED PHANTOM, COMPUTER READABLE STORAGE MEDIUM, AND CT DEVICE**

VERFAHREN ZUR BESTIMMUNG DER LÄNGSPOSITION EINES KOMBINIERTEN PHANTOMS, COMPUTERLESBARES SPEICHERMEDIUM UND CT-VORRICHTUNG

PROCÉDÉ DE DÉTERMINATION DE LA POSITION LONGITUDINALE D’UN FANTÔME COMBINÉ, SUPPORT DE STOCKAGE LISIBLE PAR ORDINATEUR ET DISPOSITIF DE TOMODENSITOMÉTRIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2021 CN 202110817910**

(43) Date of publication of application:
**24.04.2024 Bulletin 2024/17**

(73) Proprietor: **Siemens Shanghai Medical Equipment Ltd.**
**Nanhui District**
**Shanghai 201318 (CN)**

(72) Inventors:
- **LI, Tao Tao**
  **Shanghai 201315 (CN)**
- **GU, Xuan**
  **Shanghai 200122 (CN)**
- **TIAN, Yi**
  **Shanghai 200126 (CN)**
- **LOU, Yu Ting**
  **Shanghai 200052 (CN)**

(74) Representative: **Horn Kleimann Waitzhofer Schmid-Dreyer**
**Patent- und Rechtsanwälte PartG mbB**
**Theresienhöhe 12**
**80339 München (DE)**

(56) References cited:
**WO-A1-2018/081420** **US-A1- 2005 141 672**
**US-A1- 2012 076 259**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 355 216 B1

## Description

### TECHNICAL FIELD

The present invention relates to a method for determining a longitudinal position of a combined phantom.

### BACKGROUND

When a CT manufacturer releases a new software version, all on-site systems will be upgraded to this version. If some changes need tuning, all the affected systems need on-site services, which causes a heavy workload to service engineers. One solution for this is to instruct a customer to place a phantom on a correct holder and automatically align the phantom.

In addition, on a production line, the phantom is automatically aligned in a Y direction, but needs to be manually aligned in a Z direction.

US 2005/141672 A1 relates to a phantom assembly that comprises a plurality of the phantoms and an x-ray radiation from an x-ray CT device inserted in through-holes.

WO 2018/081420 A1 relates to phantoms for the calibration and quality assurance of radiation therapy systems, including biologically-guided radiation therapy systems, which deliver doses of radiation in response to real-time detected PET lines-of-response (LORs). The phantoms disclosed herein are modular phantoms that comprise a cylindrical housing with a plurality of disks stacked within the housing and a plurality of radiographic films. Some disks have a background region and a target region, where the rate of positron emissions of the target region is higher than for the background region. The disks may be arranged and rearranged within the housing to attain a desired target region shape.

US 2012/076259 A1 relates to the improving of the accuracy of a computed tomography (CT) process. This can comprise operably coupling at least one calibration phantom to a CT scan table and then, during a CT scan of an object that is disposed on the scan table, also gathering calibration information using that calibration phantom(s). By one approach, this calibration phantom can comprise one or more annular-shaped members. When using a plurality of annular-shaped members, at least some of the annular-shaped members can be disposed concentrically with one another. By one approach, in lieu of the foregoing or in combination therewith, this calibration phantom can comprise one or more pins and/or spherically-shaped members (and/or other shapes of geometric interest). If desired, such spherically-shaped members can be combined with the aforementioned pins.

### SUMMARY

In view of this, the present invention provides a method for determining a longitudinal position of a combined phantom, a computer readable storage medium, and a CT device.

According to a first aspect of the present invention, a method of determining a longitudinal position of a combined phantom is provided and includes: scanning the combined phantom to obtain a topogram thereof, the combined phantom being fixed to a table top and protruding beyond the table top, the combined phantom being axially parallel to the longitudinal direction, the combined phantom including a plurality of phantoms and one of which being a slice phantom; identifying the plurality of phantoms on the topogram; and using an axial center of the slice phantom as the longitudinal position of the combined phantom.

In an embodiment, the identifying the plurality of phantoms on the topogram includes: for each reading i, calculating a channel quantity Ci greater than an air HU value, $0 \leq i \leq N-1$ and N being a total quantity of readings; smoothing a curve of the channel quantity Ci; using a phantom length between a start position of a non-air part greater than a minimum phantom radial size and a radial size change portion as a measured length of a current phantom; and if the measured length of the current phantom is close to a length of the current phantom, identifying the current phantom.

In an embodiment, the smoothing a curve of the channel quantity $C_i$ includes: determining that adjacent readings with close channel quantities belong to the same phantom; and averaging the channel quantities of the same phantom.

In an embodiment, the channel quantities being close refers to: $|C_i - C_{i+1}| < T_C$
where $T_C$ is a positive integer threshold.

In an embodiment, $T_C = 4$.

In an embodiment, the measured length of the current phantom being close to the length of the current phantom refers to: $|L_j - L_{j,default}| < T_L$
where $L_j$ is a measured length of a phantom j, $L_{j,default}$ is a length of the phantom j, and $T_L$ is a positive threshold.

In an embodiment, $T_L = 2mm.$

In an embodiment, the longitudinal direction is a direction in which the table top enters and exits a computed tomography device.

According to a second aspect of the present invention, a computer readable storage medium is provided, on which a computer program is stored, and the program is executed by a processor to implement the method as described above.

According to a third aspect of the present invention, a computed tomography device is provided and includes a memory, a processor, and a computer program stored in the memory and executed by the processor, the processor implementing the method as described above when executing the program.

According to the method for determining a longitudinal position of a combined phantom, the computer readable storage medium, and the CT device in the present invention, the longitudinal position is determined by using the topogram of the combined phantom, and a phantom position can be quickly and automatically aligned.

## BRIEF DESCRIPTION OF THE DRAWINGS

To enable a person of ordinary skill in the art to understand the foregoing and other features and advantages of the present invention more clearly, exemplary embodiments according to the present invention are described in detail below with reference to the accompany drawings. In the accompany drawings:

FIG. 1 is a schematic flowchart of a method for determining a longitudinal position of a combined phantom according to an embodiment of the present invention.
FIG. 2 is a schematic diagram of a topogram according to an embodiment of the present invention.
FIG. 3 is a schematic flowchart of identifying a plurality of phantoms on a topogram according to an embodiment of the present invention.
FIG. 4 is a curve of a reading with regard to a channel quantity greater than an air HU value according to an embodiment of the present invention.
FIG. 5 is a schematic flowchart of smoothing a curve of a channel quantity according to an embodiment of the present invention.

In the accompany drawings, reference numerals used are as follows:

| | | | |
|---|---|---|---|
| 100 | Method | 108 | Slice phantom |
| S102, S104, S106, S108, S110, S112, S114, S116, and S118 | Steps | 110 | Water phantom |
| 102 | Combined phantom | 112 | Holder |
| 104 | Focus alignment phantom | 114 | Table top |
| 106 | High-resolution phantom | | |

## DETAILED DESCRIPTION

To make the objective, technical solutions, and advantages of the present invention clearer, the present invention is further described in detail below by using embodiments.

FIG. 1 is a schematic flowchart of a method 100 for determining a longitudinal position of a combined phantom according to an embodiment of the present invention, and FIG. 2 is a schematic diagram of a topogram 116 according to an embodiment of the present invention. As shown in FIG. 1 and FIG. 2, the method 100 for determining a longitudinal position of a combined phantom includes step S102, step S104, and step S106.

Step S102: Scan a combined phantom 102 to obtain a topogram 116 thereof. The combined phantom 102 is fixed to a table top 114, for example, by using a holder 112 and protrudes beyond the table top 114. Therefore, it can be seen from the topogram 116 that air is around the combined phantom 102. It should be noted that in the present invention, the so-called longitudinal direction is a direction in which the table top enters and exits a computed tomography device, that is, a direction Z. The combined phantom 102 is axially parallel to the longitudinal direction, and includes a plurality of phantoms, one of which is a slice phantom 108. In this embodiment, the combined phantom 102 includes a focus alignment phantom 104, a high-resolution phantom 106, the slice phantom 108, and a water phantom 110.

Step S104: Identify the foregoing phantoms on the topogram 116. FIG. 3 is a schematic flowchart of identifying a plurality of phantoms on a topogram according to an embodiment of the present invention. As shown in FIG. 3, in this embodiment, step S104 may include step S108, step S110, step S112, and step S114.

Step S108: Calculate, for each reading i, a channel quantity $C_i$ greater than an air HU value, $0 \le i \le N - 1$ and $N$ being a total quantity of readings. FIG. 4 is a curve of a reading with regard to a channel quantity $C_i$ greater than an air HU value according to an embodiment of the present invention.

Step S110: Smooth a curve of the channel quantity $C_i$.

Step S112: Use a phantom length between a start position of a non-air part greater than a minimum phantom radial size and a radial size change portion as a measured length of a current phantom.

Step S114: Identify the current phantom if the measured length of the current phantom is close to a length of the current phantom.

FIG. 5 is a schematic flowchart of smoothing a curve of a channel quantity $C_i$ according to an embodiment of the present invention. As shown in FIG. 5, step S110 of smoothing the curve of the channel quantity $C_i$ includes step S116 and step S118.

Step S116: Determine that adjacent readings with close channel quantities belong to the same phantom. The channel quantities being close may be represented as:

$$|C_i - C_{i+1}| < T_C$$

where $T_C$ is a positive integer threshold. In this embodiment, $T_C = 4$.

Step S118: Average the channel quantities of the same phantom.

In step S114, the measured length of the current phantom being close to the length of the current phantom refers to:

$$|L_j - L_{j,default}| < T_L$$

where $L_j$ is a measured length of a phantom j, $L_{j,default}$ is a length of the phantom j, and $T_L$ is a positive threshold. In this embodiment, $T_L = 2mm$.

As shown in FIG. 1, after step S104 of identifying the phantom on the topogram 116, in step S106, an axial center of the slice phantom 108 is used as the longitudinal position of the combined phantom 102.

After the longitudinal position of the combined phantom is calculated, a position of another phantom may be checked according to the position and a default distance of each phantom in a configuration table. If all phantoms can cover a longitudinal range of scanning, the longitudinal position of the phantom is confirmed.

According to another aspect of the present invention, a computer readable storage medium is provided, storing a computer program, and the program, when executed by a processor, implementing the method 100 for determining a longitudinal position of a combined phantom.

According to still another aspect of the present invention, a computed tomography device is provided, including a memory, a processor, and a computer program stored on the memory and executable on the processor, the processor implementing the method 100 for determining a longitudinal position of a combined phantom when executing the program.

According to the method for determining a longitudinal position of a combined phantom, the computer readable storage medium, and the CT device in the present invention, the longitudinal position is determined by using the topogram of the combined phantom, which can quickly and automatically align a phantom position.

The foregoing descriptions are merely preferred embodiments of the present invention, but are not intended to limit the present invention.

## Claims

**1.** A method for determining a longitudinal position of a combined phantom (102) in a computed tomography device, comprising:

scanning the combined phantom (102) to obtain a topogram (116) thereof, the combined phantom (102) being fixed to a table top (114) and protruding beyond the table top (114), the combined phantom (102) being axially parallel to the longitudinal direction, the combined phantom (102) comprising a plurality of phantoms and one of which being a slice phantom (108);
identifying the plurality of phantoms on the topogram (116); and
using an axial center of the slice phantom (108) as the longitudinal position of the combined phantom (102).

**2.** The method according to claim 1, wherein the identifying the plurality of phantoms on the topogram (116) comprises:

for each reading i, calculating a channel quantity Ci greater than an air HU value, $0 \leq i \leq N-1$ and N being a total quantity of readings;
smoothing a curve of the channel quantity Ci;
using a phantom length between a start position of a non-air part greater than a minimum phantom radial size and a radial size change portion as a measured length of a current phantom; and
if the measured length of the current phantom is close to a length of the current phantom, identifying the current phantom.

3. The method according to claim 2, wherein the smoothing a curve of the channel quantity Ci comprises:

determining that adjacent readings with close channel quantities belong to the same phantom; and averaging the channel quantities of the same phantom.

4. The method according to claim 3, wherein the channel quantities being close refers to:

$$|Ci\text{-}C_{(i+1)}|<T_C$$

wherein $T_C$ is a positive integer threshold.

5. The method according to claim 4, wherein Tc=4.

6. The method according to claim 2, wherein the measured length of the current phantom being close to the length of the current phantom refers to:

$$|Lj\text{-}L_{j,default}|<T_L$$

wherein $L_j$ is a measured length of a phantom j, $L_{j,default}$ is a length of the phantom j, and $T_L$ is a positive threshold.

7. The method according to claim 6, wherein $T_L$=2mm.

8. The method according to claim 1, wherein the longitudinal direction is a direction in which the table top enters and exits the computed tomography device.

9. A computer readable storage medium that stores a computer program, the program being executed by a processor to implement the method according to any one of claims 1 to 8.

10. A computed tomography device, comprising a memory, a processor, and a computer program stored in the memory and executed by the processor, the processor implementing the method according to any one of claims 1 to 8 when executing the program.

## Patentansprüche

1. Verfahren zur Bestimmung der Längsposition eines kombinierten Phantoms (102) in einer Computertomographievorrichtung, umfassend:

Scannen des kombinierten Phantoms (102), um ein Topogramm (116) davon zu erhalten, wobei das kombinierte Phantom (102) an einer Tischplatte (114) befestigt ist und über die Tischplatte (114) hinausragt, wobei das kombinierte Phantom (102) achsparallel zur Längsrichtung ist, wobei das kombinierte Phantom (102) eine Mehrzahl von Phantomen umfasst und eines davon ein Scheibenphantom (108) ist;
Identifizieren der Mehrzahl von Phantomen auf dem Topogramm (116); und
Verwenden einer axialen Mitte des Scheibenphantoms (108) als Längsposition des kombinierten Phantoms (102).

2. Verfahren nach Anspruch 1, wobei das Identifizieren der Mehrzahl von Phantomen auf dem Topogramm (116) umfasst:

für jeden Ablesewert i, Berechnen einer Kanalmenge Ci, die größer als ein Luft-HU-Wert ist, wobei 0≤i≤N-1 und N eine Gesamtmenge von Ablesewerten ist;
Glätten einer Kurve der Kanalmenge Ci;
Verwenden einer Phantomlänge zwischen einer Startposition eines Nicht-Luft-Teils, die größer als eine minimale radiale Größe des Phantoms ist, und einem Abschnitt der Änderung der radialen Größe als eine gemessene Länge eines aktuellen Phantoms; und

wenn die gemessene Länge des aktuellen Phantoms nahe an einer Länge des aktuellen Phantoms liegt, Identifizieren des aktuellen Phantoms.

3. Verfahren nach Anspruch 2, wobei das Glätten einer Kurve der Kanalmenge Ci umfasst:

   Feststellung, dass benachbarte Ablesewerte mit nahe beieinander liegenden Kanalmengen zu demselben Phantom gehören; und
   Bilden des Mittelwerts der Kanalmengen desselben Phantoms.

4. Verfahren nach Anspruch 3, wobei sich die Tatsache, dass die Kanalmengen nahe beieinander liegen, auf folgendes bezieht:

$$|Ci-C_{(i+1)}|<T_C$$

   wobei Tc ein positiver ganzzahliger Schwellenwert ist.

5. Verfahren gemäß Anspruch 4, wobei Tc=4.

6. Verfahren nach Anspruch 2, wobei sich die Tatsache, dass die gemessene Länge des aktuellen Phantoms nahe an einer Länge des aktuellen Phantoms liegt, auf folgendes bezieht:

$$|Lj-L_{j,default}|<T_L$$

   wobei $L_j$ eine gemessene Länge eines Phantoms j ist, $L_{j,default}$ eine Länge des Phantoms j ist und $T_L$ ein positiver Schwellenwert ist.

7. Verfahren gemäß Anspruch 6, wobei $T_L$=2 mm.

8. Verfahren nach Anspruch 1, wobei die Längsrichtung eine Richtung ist, in der die Tischplatte in die Computertomographievorrichtung eintritt und daraus austritt.

9. Computerlesbares Speichermedium, das ein Computerprogramm speichert, wobei das Programm von einem Prozessor ausgeführt wird, um das Verfahren nach einem der Ansprüche 1 bis 8 zu implementieren.

10. Computertomographievorrichtung, umfassend einen Speicher, einen Prozessor und ein Computerprogramm, das im Speicher gespeichert ist und vom Prozessor ausgeführt wird, wobei der Prozessor das Verfahren nach einem der Ansprüche 1 bis 8 implementiert, wenn er das Programm ausführt.

## Revendications

1. Procédé de détermination d'une position longitudinale d'un fantôme combiné (102) dans un dispositif de tomodensitométrie, comportant :

   un balayage du fantôme combiné (102) pour obtenir un topogramme (116) de celui-ci, le fantôme combiné (102) étant fixé à un plateau (114) de table et faisant saillie au-delà du plateau (114) de table, le fantôme combiné (102) étant axialement parallèle à la direction longitudinale, le fantôme combiné (102) comportant une pluralité de fantômes dont l'un est un fantôme (108) de tranche ;
   l'identification de la pluralité de fantômes sur le topogramme (116) ; et
   l'utilisation d'un centre axial du fantôme (108) de tranche en tant que position longitudinale du fantôme combiné (102).

2. Procédé selon la revendication 1, l'identification de la pluralité de fantômes sur le topogramme (116) comportant :

   pour chaque indication i, le calcul d'une grandeur Ci de canal supérieure à une valeur HU de l'air, 0≤ i≤ N-1 et N étant une quantité totale d'indications ;
   le lissage d'une courbe de la grandeur Ci de canal ;

l'utilisation d'une longueur de fantôme entre une position de début d'un partie autre que l'air supérieure à une taille radiale minimum de fantôme et une partie de variation de taille radiale en tant que longueur mesurée d'un fantôme actuel ; et

si la longueur mesurée du fantôme actuel est proche d'une longueur du fantôme actuel, l'identification du fantôme actuel.

**3.** Procédé selon la revendication 2, le lissage d'une courbe de la grandeur Ci de canal comportant :

le fait de déterminer que des indications adjacentes dont les grandeurs de canal sont proches appartiennent au même fantôme ; et

le fait de prendre la moyenne des grandeurs de canal du même fantôme.

**4.** Procédé selon la revendication 3, le fait que les grandeurs de canal soient proches faisant référence à :

$$|Ci-C_{(i+1)}| < T_C$$

où Tc est un seuil entier positif.

**5.** Procédé selon la revendication 4, où Tc=4.

**6.** Procédé selon la revendication 2, le fait que la longueur mesurée du fantôme actuel soit proche de la longueur du fantôme actuel faisant référence à :

$$|Lj-L_{j,default}| < T_L$$

où $L_j$ est une longueur mesurée d'un fantôme j, $L_{j,default}$ est une longueur du fantôme j, et $T_L$ est un seuil positif.

**7.** Procédé selon la revendication 6, où $T_L$=2 mm.

**8.** Procédé selon la revendication 1, la direction longitudinale étant une direction dans laquelle le plateau de table entre dans le dispositif de tomodensitométrie et en sort.

**9.** Support de stockage lisible par ordinateur qui conserve un programme informatique, le programme étant exécuté par un processeur pour mettre en œuvre le procédé selon l'une quelconque des revendications 1 à 8.

**10.** Dispositif de tomodensitométrie, comportant une mémoire, un processeur et un programme informatique conservé dans la mémoire et exécuté par le processeur, le processeur mettant en œuvre le procédé selon l'une quelconque des revendications 1 à 8 lorsqu'il exécute le programme.

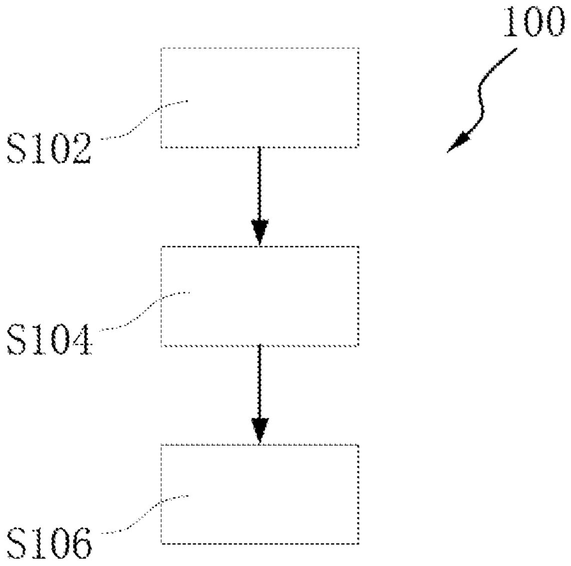


FIG. 1

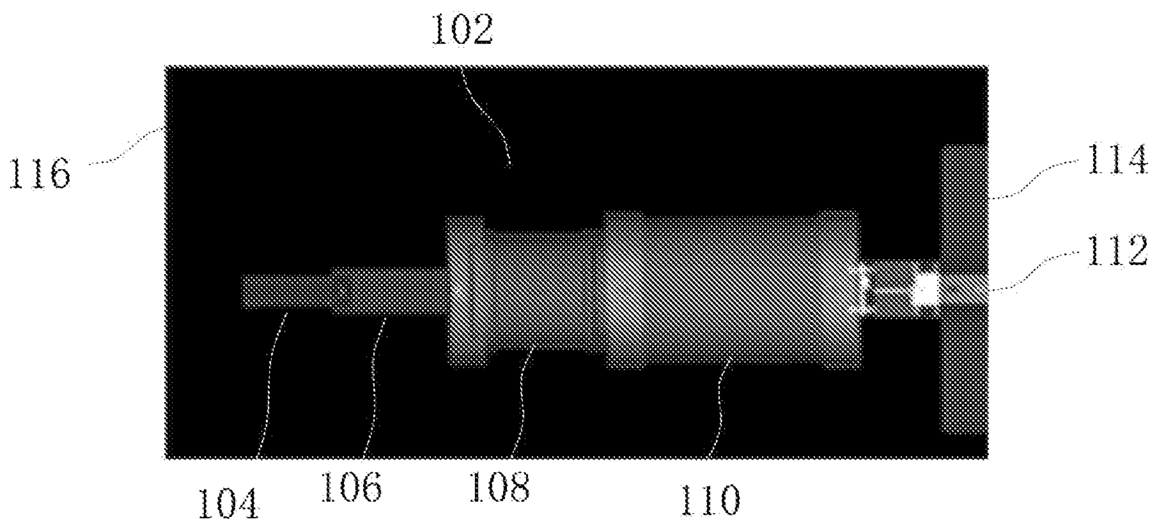


FIG. 2

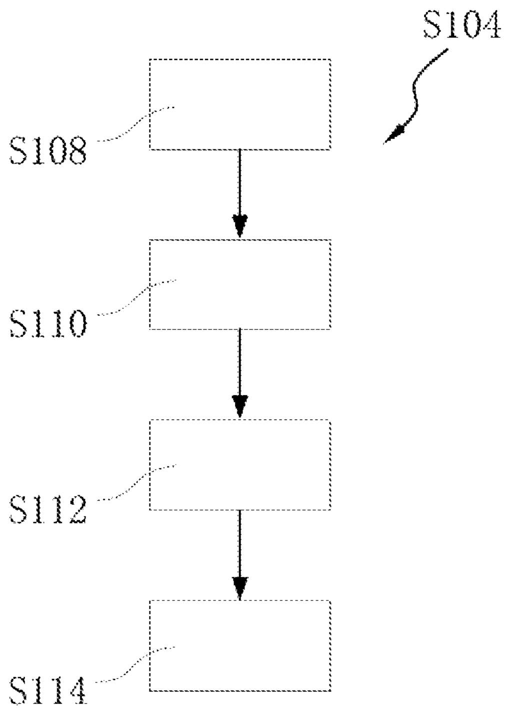
S104
S108
S110
S112
S114

FIG. 3

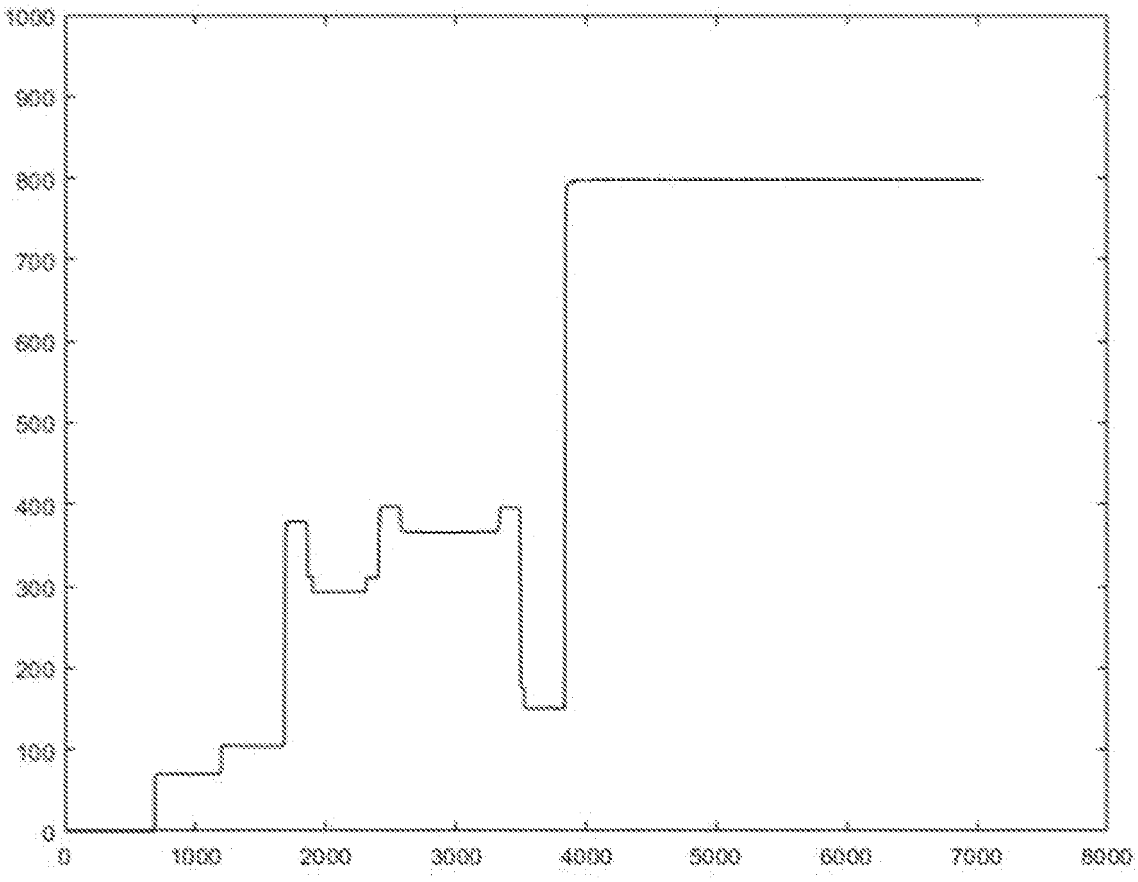
1000
900
800
700
600
500
400
300
200
100
0
0
1000
2000
3000
4000
5000
6000
7000
8000

FIG. 4

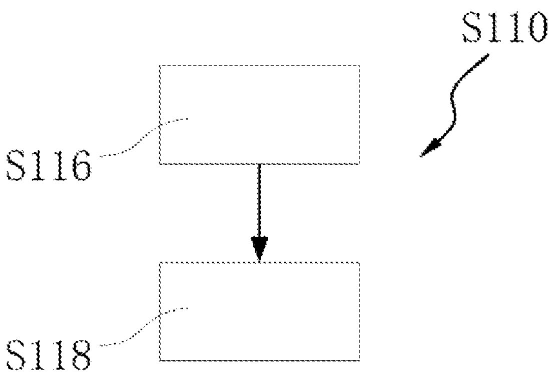


FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2005141672 A1
- WO 2018081420 A1
- US 2012076259 A1